(19) Europäisches Patentamt
European Patent Office
Office européen des brevets

(11) Publication number: **0 161 769**
**B1**

(12) **EUROPEAN PATENT SPECIFICATION**

(45) Date of publication of the patent specification: **28.10.87**

(51) Int. Cl.[4]: **C 07 C 149/24**, C 07 C 103/38, C 07 C 153/01

(21) Application number: **85302185.5**

(22) Date of filing: **28.03.85**

(54) **Enkephalinase inhibitors.**

(30) Priority: **02.04.84 US 595765**

(43) Date of publication of application: **21.11.85 Bulletin 85/47**

(45) Publication of the grant of the patent: **28.10.87 Bulletin 87/44**

(84) *Designated Contracting States:* **AT BE CH DE FR GB IT LI LU NL SE**

(56) References cited:
**EP - A - 0 038 758**
**GB - A - 2 076 809**

(73) Proprietor: **E.R. Squibb & Sons, Inc., Lawrenceville-Princeton Road, Princeton, N.J. 08540 (US)**

(72) Inventor: **Delaney, Norma G., 137 Cherry Brook Drive, Princeton New Jersey (US)**
Inventor: **Gordon, Eric M., 126 Laning Avenue, Pennington New Jersey (US)**

(74) Representative: **Thomas, Roger Tamlyn et al, D. Young & Co. 10 Staple Inn, London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to new compounds having the formula

$$\begin{array}{l} \quad R_2 \quad\quad O\ R_3 \quad\quad\quad O \\ \quad | \quad\quad\quad \| \ \ | \quad\quad\quad\quad \| \\ R_1\text{-S-CH}_2\text{-CH-NH-C-CH-(CH}_2)_n\text{-C-}R_4 \end{array} \quad I$$

and pharmaceutically acceptable salts thereof, which are useful analgesic agents. In formula I, and throughout the specification, the symbols are as defined below.

$R_1$ is hydrogen or $R_5\text{-}\overset{\overset{O}{\|}}{C}\text{-}$, wherein $R_5$ is alkyl, aryl, arylalkyl, heteroaryl, or (heteroaryl)alkyl;

$R_2$ and $R_3$ are each independently hydrogen, alkyl, (cycloalkyl)alkyl, aryl, arylalkyl, heteroaryl, or (heteroaryl)alkyl;

$R_4$ is hydroxy, alkoxy, arylalkoxy, (heteroaryl)-alkoxy, (substituted alkyl)oxy, or $\text{-NR}_6R_7$, wherein $R_6$ and $R_7$ are each independently hydrogen, alkyl, substituted alkyl, aryl, arylalkyl, or (heteroaryl)alkyl; and

n is an integer of 1 to 15.

The terms «alkyl», «alkoxy», and «alkylthio», as used throughout the specification either individually or as part of a larger group, refer to straight and branched-chain groups having 1 to 7 carbon atoms.

The term «cycloalkyl» as used throughout the specification, either individually or as part of a larger group, refers to cycloalkyl groups having 3, 4, 5, 6 or 7 carbon atoms.

The term «aryl» as used throughout the specification, either individually or as part of a larger group, refers to phenyl and phenyl substituted with 1, 2 or 3 alkyl, alkoxy, alkylthio, hydroxy, chlorine, bromine, fluorine, amino, alkylamino, dialkylamino, nitro or trifluoromethyl groups.

The term «heteroaryl», as used throughout the specification, either individually or as part of a larger group, refers to 2- or 3-furanyl, 2- or 3-thienyl, 2-, 3- or 4-pyridinyl, 4-imidazolyl, and 3-indolyl.

The term «substituted alkyl», as used throughout the specification, either individually or as part of a larger group, refers to alkyl groups substituted with one, or more (preferably one), hydroxy or $NY_1Y_2$ groups, wherein $Y_1$ and $Y_2$ ate the same or different and each is hydrogen or alkyl, $Y_1$ is hydrogen and $Y_2$ is aryl, or $Y_1$ and $Y_2$ together with the nitrogen atom to which they are attached form a heterocyclic group having the formula

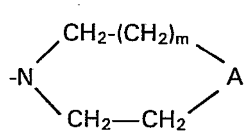

and A is CH-Q, oxygen, or N-Q, Q is hydrogen or alkyl and m is 0 or 1.

Greenberg et al. in United States Patent 4 401 677 disclose that various mercaptoalkanoyl β-amino acids are useful analgesic agents due to their enkephalinase inhibition activity.

Ondetti et al. in United States Patent 4 053 651 disclose that various mercaptoalkanoyl and acylmercaptoalkanoyl β-aminoacids are useful hypotensive agents due to their angiotensin converting enzyme inhibition activity.

Roques et al. (Nature, Vol. 288, Nov. 1980, p. 286-288) disclose that thiorphan, [(D,L)-3-mercapto-2-benzylpropanoyl]glycine, is an inhibitor of enkephalinase <u>in vitro</u> in nanomolar concentrations and <u>in vivo</u> after either intracerebroventricular or systemic administration.

Roques et al. (Proc. Natl. Acad. Sci. (U.S.A.) Vol. 80, 1983, p. 3178-3182) disclose thiorphan and its retro-inverso isomer, and their activity as inhibitors of enkephalinase.

Roques et al. in European Patent Application 38 758 disclose various β-amino acid derivatives including mercaptoalkanoyl and acyl-mercaptoalkanoyl derivatives as possessing enkephalinase inhibition activity.

Mumford et al. (Biochemical and Biophysical Research Comm., Vol. 109, No. 4, 1982, p. 1303-1309) disclose that various substituted N-carboxymethyl dipeptides including those having a terminal β-alanine group possess enkephalinase inhibition activity.

Berger et al. in European Patent Application 54 862 disclose enkephalinase inhibition peptides having the structural formula

$$\begin{array}{l} \quad\quad\quad\quad R_c \quad\quad\quad\quad\quad\quad R_d \\ \quad\quad\quad\quad | \quad\quad\quad\quad\quad\quad\quad | \\ R_a\text{-HC—N—X-CO-NH-CH-(CH}_2)_y\text{-CO-}R_e \\ \quad\quad | \\ \quad\quad R_b \end{array}$$

wherein $R_b$ is a carboxylic or phosphonic acid or ester and y is 0, 1, 2 or 3.

Cushman et al. (Biochemistry, Vol. 16, No. 25, 1977, p. 5484-5491) disclose various carboxyalkanoyl and mercaptoalkanoyl amino acids as angiotensin converting enzyme inhibitors. Among the compounds disclosed is

$$\begin{array}{l} \quad\quad\quad\quad\quad\quad\quad O \\ \quad\quad\quad\quad\quad\quad\quad \| \\ \text{HS-(CH}_2)_2\text{-C-NH-(CH}_2)_2\text{-COOH .} \end{array}$$

Sundeen et al. in United States Patents 4 235 885 and 4 297 275 disclose mammalian collagenase inhibitors including mercaptoalkanoyl and acylmercaptoalkanoyl compounds having the structural formula

$$\begin{array}{l} \quad (H_3C)_2\text{-CH-CH}_2 \quad\quad O \quad\quad\quad\quad\quad\quad R_g\ \ O \\ \quad\quad\quad\quad\quad\quad | \quad\quad\quad\quad \| \quad\quad\quad\quad\quad\quad\quad | \ \ \| \\ R_f\text{-S-CH}_2\text{-CH——C-NH-(CH}_2)_m\text{-CH-C-}R_h \end{array}$$

wherein m is zero or an integer from 1 to 9, $R_g$ can be, <u>inter alia</u>, hydrogen, and $R_h$ can be <u>inter alia</u>, hydroxy and amino.

Sundeen et al. in United States Patent 4 327 111 disclose mammalian collagenase inhibitors including mercaptoalkanoyl and acylmercaptoalkanoyl compounds of the formula

$$\begin{array}{l} \quad\quad\quad R_j \quad\quad O \quad\quad\quad\quad\quad\quad O \\ \quad\quad\quad | \quad\quad\quad \| \quad\quad\quad\quad\quad\quad \| \\ R_i\text{-}S\text{-}CH_2\text{-}CH\text{---}C\text{-}NH\text{-}(CH_2)_n\text{-}C\text{-}R_k \end{array}$$

wherein $R_k$ is hydrogen, alkyl or aryl, n is an integer from 1 to 20, and $R_j$ is alkyl of 3 to 8 carbons, cycloalkyl of 3 to 7 carbons, aryl, or arylalkyl.

The compounds of this invention can be administered to a mammalian species as an analgesic agent due to their ability to inhibit enkephalinase activity. While not limiting the scope of the invention to a specific theory or mechanism of action, it has been suggested that the andogenous opioid pentapeptides, [Met$^5$]-enkephalin(Tyr-Gly-Gly-Phe-Met) and [Leu$^5$]enkephalin(Tyr-Gly-Gly-Phe-Leu), are neurotransmitters involved in central pain mediation (Hughes et al., Nature, Vol. 258, December 1975, p. 577-579) and that these endogenous opioid peptides are functionally inactivated by cleavage of their Gly$^3$-Phe$^4$ peptide bonds by a specific peptidase, enkephalinase presumed to be specifically located at nerve terminals in the brain where enkephalins are released (Malfroy et al., Nature, Vol. 276, November 1978, p. 523-526). Specific inhibitors of this enkephalinase enhance the recovery of endogenous enkephalins released from isolated brain slices (Patey et al., Science, Vol. 212, June 1981, p. 1153-1155) and cause analgesia in mice that is reversed by the opioid antagonist naloxone (Roques et al., supra). In addition to analgesia, other pharmaceutical actions such as antitussive or antidiarrheal activities may result from prolonging the action of the body's natural opiates released from peripheral as well as central sites.

By the administration of a composition containing one or a combination of compounds of this invention, pain is alleviated in the mammalian host. A single dose, or preferably two to four divided daily doses, provided on a basis of about 0.1 to about 100 mg per kilogram of body weight per day, preferably about 1 to about 50 mg per kilogram per day, produces the desired analgesic activity. The composition is preferably administered orally but parenteral routes such as subcutaneous can also be employed.

In particular, the compounds of formula I, while possessing potent enkephalinase inhibition activity, are far less active as angiotensin converting enzyme inhibitors than the corresponding α-amino acid compounds of the prior art. Administration of the compounds of formula I will produce a selective analgesic effect not possible with the α-amino acid compounds of the prior art.

The compounds of this invention can also be formulated in combination with an aminopeptidase inhibitor for the treatment of pain in mammalian species. Aminopeptidase inhibitors are known in the art; see, for example, Wagner et al., Journal of Neurochemistry, Vol. 37, p. 709-713 (1981).

The compounds of this invention can be prepared by first reacting a compound having the formula

$$\begin{array}{l} \quad\quad\quad\quad R_2 \\ \quad\quad\quad\quad | \\ HO\text{-}CH_2\text{-}CH\text{-}NH_2 \end{array} \qquad \text{II}$$

with a compound having the formula

$$\begin{array}{l} \; O \; R_3 \quad\quad\quad O \\ \; \| \;\; | \quad\quad\quad\quad \| \\ HO\text{-}C\text{-}CH\text{-}(CH_2)_n\text{-}C\text{-}R_4' \end{array} \qquad \text{III}$$

wherein $R_4'$ is alkoxy, to obtain a compound having the formula

$$\begin{array}{l} \quad\quad\quad\quad R_2 \quad\quad O \; R_3 \quad\quad\quad O \\ \quad\quad\quad\quad | \quad\quad\quad \| \;\; | \quad\quad\quad\quad \| \\ HO\text{-}CH_2\text{-}CH\text{-}NH\text{-}C\text{-}CH\text{-}(CH_2)_n\text{-}C\text{-}R_4' \end{array} \qquad \text{IV}$$

The reaction is preferably run in the presence of a coupling agent such as dicyclohexylcarbodiimide. Alternatively, the carboxylic acid of formula III can be activated by formation of its mixed anhydride, symmetrical anhydride, acid halide, active ester or the use of Woodward reagent K, N-ethoxycarbonyl-2-ethoxy-1,2-dihydroquinoline or the like. For a review of the methods of acylation, see Methoden der Organischen Chemie (Houben-Weyl), Vol. XV, part II, page 1 et seq. (1974). Preferably, the acid halide, especially the acid chloride, of formula III is employed.

Reaction of a compound of formula IV with an acylthiol compound having the formula

$$\begin{array}{l} \quad\; O \\ \quad\; \| \\ R_5\text{-}C\text{-}SH \end{array} \qquad \text{V}$$

yields a compound having the formula

$$\begin{array}{l} \; O \quad\quad\quad R_2 \quad\quad O \; R_3 \quad\quad\quad O \\ \; \| \quad\quad\quad\; | \quad\quad\quad \| \;\; | \quad\quad\quad\quad \| \\ R_5C\text{-}S\text{-}CH_2\text{-}CH\text{-}NH\text{-}C\text{-}CH\text{-}(CH_2)_n\text{-}C\text{-}R_4' \end{array} \qquad \text{VI}$$

i.e., compounds of formula I wherein $R_1$ is $R_5$-C(=O)- and $R_4'$ is alkoxy. The reaction is a Mitsunobu reaction and is run in the presence of triphenylphosphine and a dialkyl azodicarboxylate.

Subjecting a compound of formula VI to conventional hydrolysis yields the corresponding products of formula I having the formula

$$\begin{array}{l} \quad\quad\quad\quad R_2 \quad\quad O \; R_3 \quad\quad\quad O \\ \quad\quad\quad\quad | \quad\quad\quad \| \;\; | \quad\quad\quad\quad \| \\ HS\text{-}CH_2\text{-}CH\text{-}NH\text{-}C\text{-}CH\text{-}(CH_2)_n\text{-}C\text{-}OH \end{array} \qquad \text{VII}$$

i.e., products of formula I wherein $R_1$ is hydrogen and $R_4$ is hydroxy.

Those products of formula I wherein $R_1$ is hydrogen and $R_4$ is alkoxy can be prepared by selectively saponifying a corresponding S-acylated compound of formula VI.

Alternatively, those products of formula I wherein $R_1$ is hydrogen and $R_4$ is alkoxy can be prepared by esterification of the corresponding acid of formula VII with the appropriate alcohol using dicyclohexylcarbodiimide and dimethylaminopyridine, or an acid catalyst such as sulfuric acid or by reaction with the appropriate diazo compound.

Those products of formula I wherein $R_1$ is $R_5-\overset{O}{\overset{\|}{C}}-$ and $R_4$ is hydroxy can be prepared by acylation of the corresponding compound of formula VII with the appropriate acyl halide.

Those products of formula I wherein $R_4$ is $-NR_6R_7$ can be prepared by reacting the corresponding product of formula I wherein $R_4$ is hydroxy with an amine having the formula

$$HNR_6R_7 \qquad \text{VIII}$$

or if $R_6$ and $R_7$ are both kydrogen, with a methanolic solution of ammonia. The reaction can be accomplished using known amide bond forming procedures. For example, the reaction can be run in the presence of a coupling agent such as dicyclohexylcarbodiimide, or the acid of formula I can be activated by formation of its mixed anhydride, symmetrical anhydride, acid halide (preferably acid chloride) or acid ester, or by the use of Woodward reagent K, N-ethoxycarbonyl-2-ethoxy-1,2-dihydroquinoline, N,N'-carbonyldiimidazole or the like. A review of these methods can be found in Methoden der Organischen Chemie (Houben-Weyl), Vol. XV, part II, page 1 et seq. (1974).

The compounds of formula I wherein $R_4$ is hydroxy form basic salts with a variety of inorganic and organic bases. The pharmaceutically acceptable salts include alkali metal salts such as sodium or potassium, alkaline earth metal salts such as calcium or magnesium, and salts derived from amino acids such as arginine, lysine, etc. The salts can be prepared by reacting the acid form of the compound, i.e., $R_4$ is hydroxy, with an equivalent of the base supplying the desired basic ion in a medium in which the salt precipitates or in aqueous medium and then lyophilizing.

Additionally, the compounds of formula I containing an amine group form acid-addition salts with a variety of inorganic and organic acids. The pharmaceutically acceptable salts include, for example, the hydrohalides, e.g., hydrochloride, hydrobromide, etc., sulfate, phosphate, nitrate, arylsulfonates, e.g., camphorsulfonate, benzenesulfonate, toluenesulfonate, etc., citrate, ascorbate, maleate, fumarate, pamoate, acetate, tartrate, salicylate and the like. It is frequently convenient to isolate the compound by forming the acid salt and precipitating in a medium in which it is insoluble. The bases also form quaternary ammonium salts with quaternizing agents which are acceptable for pharmaceutical use., e.g., lower alkyl halides such as methyl chloride, methyl bromide, ethyl chloride, etc., lower alkyl sulfates such as methyl sulfate, ethyl sulfate, etc., monocyclic aryl (lower alkyl) halides and sulfates such as benzyl choride, benzyl sulfate, etc. This is accomplished by reactimg the base with the alkyl halide, sulfate or the like.

The following examples are specific embodiments of this invention.

*Example 1*

*6-[[1-(Mercatomethyl)-2-phenylethyl]amino]-6-oxohexanoic acid*

A) *6-[[1-(Hydroxymethyl)-2-phenylethyl]amino]-6-oxohexanoic acid, ethyl ester*

Diisopropylethylamine (4.36 ml, 25 mmol) was added dropwise to a stirred suspension of 2-amino-3-phenyl-1-propanol hydrochloride (4.69 g, 25 mmol) in dichloromethane (100 ml) at 0°C. After waiting one hour (all material was not in solution), a solution of adipic acid monoethyl ester (4.36 g, 25 mmol) in 50 ml of dichloromethane was added, followed by the dropwise addition of a suspension of dicyclohexylcarbodiimide (5.16 g, 25 mmol) in 100 ml of dichloromethane, over 5 minutes. The reaction mixture was then removed from the ice bath and left to stir at room temperature overnight. The dicyclohexylurea by-product was filtered off and washed with ethyl acetate. The filtrate was evaporated and the residue was triturated with ethyl acetate (500 ml) and again filtered. The filtrate was washed sequentially with 10% potassium bisulfate, water, half-saturated sodium bicarbonate, water and brine (three 50 ml portions of each), dried over sodium sulfate and concentrated in vacuo to give 7.45 g of a light yellow solid. This material was adsorbed onto a small amount of Merck silica (230-400 mesh), dried and applied to a column of ~375 g of the same silica. Elution with hexane/ethyl acetate (4:3) initially, then gradually increasing the eluent polarity to ethyl acetate and finally 10% methanol in ethyl acetate, yielded 3.17 g of a yellow mobile oil.

B) *6-[[1-[(Acetylthio)methyl]-2-phenylethyl]-amino]-6-oxohexanoic acid, ethyl ester*

Diisopropyl azodicarboxylate (2.99 ml, 15.2 mmol) was added to an efficiently stirred solution of triphenylphosphine (3.99 g, 15.2 mmol) in tetrahydrofutan (30 ml) at 0°C. The mixture was stirred at 0°C for 30 minutes, during which time a light yellow material precipitated. A solution of thiolacetic acid (1.09 ml, 15.2 mmol) and 6-[[1-(hydroxymethyl)-2-phenylethyl]amino]-6-oxohexanoic acid, ethyl ester (2.35 g, 7.6 mmol) in 35 ml of tetrahydrofuran was added over 10 minutes. The mixture was stirred for 2 hours at 0°C, then allowed to warm to room temperature and left to stir for 2.5 days. The mixture was concentrated in vacuo, and the resulting residue was taken up in 125 ml of ethyl acetate and washed with saturated sodium bicarbonate (three 30 ml portions), dried with sodium sulfate and concentrated to yield a yellow oil with a white precipitate, 11.05 g. The residue was taken up in ethyl acetate/hexane (1:1), filtered three times to remove precipitate and concentrated. The remaining yellow oil was applied to a column of ~450 g of Merck silica (230-400 mesh) and eluted with 1:1 hexane/ethyl acetate to yield 2.48 g of a white solid, melting point 63-64°C (sinters >46°C).

C) *6-[[1-(Mercaptomethyl)-2-phenylethyl]amino]-6-oxohexanoic acid*

6-[[1-[(Acetylthio)methyl]-2-phenylethyl]-amino]-6-oxohexanoic acid, ethyl ester (2.47 g, 6.76 mmol) was dissolved in cold (0°C) methanol (21 ml), and stirred under nitrogen. To this solution was added, dropwise over 10 minutes, 1N sodium hydroxide (21 ml, ~3 equiv.). The mixture was stirred at 0°C for

10 minutes, warmed to room temperature and left to stir for 3 hours. The mixture was concentrated to half volume in vacuo and the residue was diluted with water (40 ml) and then washed with chloroform (two 20 ml portions). The aqueous layer was acidified to pH ~ 1.5 with concentrated hydrochloric acid. The resulting white suspension was extracted with ethyl acetate (three 15 ml portions). These organic layers were combined, washed with water and brine (20 ml each), dried over sodium sulfate and concentrated to yeld a white solid, 1,90 g. This material was dissolved by heating in a small volume of toluene/acetic acid (4:1), applied to a column of ~ 125 g of Merck silica (230-400 mesh) and eluted with the same solvent, to yield 1.70 g of a colorless oil, which was taken up in ethyl acetate and concentrated to give 1.53 g of the title compound as a white solid, melting point 82-85°C.

Analysis Calc'd. for $C_{15}H_{21}NO_3S$:

C 60.99; H 7.17; N 4.74; S 10.85

Found: C 60.92; H 7.15; N 4.76; S 10.75.

*Example 2*

*8-[[1-(Mercaptomethyl)-2-phenylethyl]amino]-8-oxooctanoic acid, methyl ester*

A) *Suberic acid, dimethyl ester*

A solution of suberic acid (52.26 g, 300 mmol) in 900 ml of methanolic hydrogen chloride (prepared by bubbling gaseous hydrogen chloride through methanol for 15 minutes) was heated under reflux for 5 hours and then stirred at room temperature overnight. The mixture was concentrated in vacuo. The resulting residue was taken up in ethyl acetate (900 ml) and washed with 5% sodium bicarbonate (3 × 150 ml), water (3 × 125 ml) and brine (150 ml), dried over sodium sulfate and concentrated to yield 56.06 g of a light yellow liquid.

B) *Suberic acid, monomethyl ester*

To a cold (<5°C) solution of suberic acid, dimethyl ester (20.23 g, 100 mmol) in methanol (100 ml) was added, dropwise with stirring under nitrogen, 1N sodium hydroxide (100 ml, 1.0 equivalent). After addition of the sodium hydroxide, the solution was allowed to warm to room temperature and stirred for 4 hours. The mixture was then concentrated to half volume in vacuo. The residue was diluted with water (200 ml), adjusted to pH ~ 9.0 with 1N sodium hydroxide and extracted with ethyl acetate (2 × 60 ml). The aqueous layer was acidified to pH ~ 2.0 with 1N hydrochloric acid, and again extracted with ethyl acetate (3 × 60 ml). These organic layers were combined, washed with water and brine (60 ml each), dried over sodium sulfate, and concentrated in vacuo to yield 12.53 g of a mixture of mono- and diacid, as a white solid. This was dissolved in a minimal amount of methanol and adsorbed onto a small amount of Merck silica gel (230-400 mesh) and dried. This material was chromatographed in three portions. In a typical separation, approximately 5.6 g of mixture was applied to a column of 350 g of Merck silica gel and eluted with toluene/acetic acid (20:1) to yield 3.23 g of the desired monoester as a colorless liquid. The same column was used to separate a second portion of the mixture. The combined yield of monoester was 7.41 g.

C) *8-[[1-(Hydroxymethyl)-2-phenylethyl]amino]-8-oxooctanoic acid, methyl ester*

To a solution of suberic acid, monomethyl ester (6,76 g, 35.9 mmol) in ether (125 ml) was added oxalyl chloride (3.45 ml, 39.5 mmol). This mixture was cautiously treated with a catalytic amount (3 drops) of dimethylformamide, and then stirred for one hour at room temperature under nitrogen. The mixture was concentrated in vacuo, producing an oil which was dissolved in tetrahydrofuran (75 ml) and again concentrated in vacuo. The resulting residue was dissolved in dichloromethane (75 ml) and added dropwise over one hour to a cold (-5°C), stirred suspension of 2-amino-3-phenyl-1-propanol hydrochloride (8.76 g, 46.7 mmol) and diisopropylethylamine (16.26 ml, 93,4 mmol) in dichloromethane (125 ml). After stirring in the cold (-5°C) for 2.5 hours under nitrogen, the mixture was allowed to warm to room temperature and left to stir overnight. The mixture was concentrated in vacuo and the residue taken up into ethyl acetate (650 ml) and gravity filtered to remove diisopropylethylamine hydrochloride. The filtrate was washed sequentially with 10% potassium bisulfate, water, 5% sodium bicarbonate, water and brine (3 × 100 ml each). The organic layer was dried over sodium sulfate and concentrated to yield 10.94 g of a viscous yellow oil.

D) *8-[[1-(Acetylthioy)methyl]-2-phenylethyl]-amino]-8-oxooctanoic acid, methyl ester*

Diisopropyl azodicarboxylate (13.41 ml, 68.08 mmol) was added to an efficiently stirred solution of triphenylphosphine (17.86 g, 68.08 mmol) in tetrahydrofuran (200 ml) at 0°C. The mixture was stirred at 0°C for 30 minutes, during which time a light yellow precipitate resulted. A solution of thiolacetic acid (4.87 ml, 68.08 mmol) and 8-[[1-hydroxymethyl)-2-phenylethyl]amino]-8-oxooctanoic acid, methyl ester (10.94 g, 34.04 mmol) in 150 ml of tetrahydrofuran was added dropwise over 10 minutes. The turbid green mixture was stirred for 1 hour at 0°C, allowed to warm to room temperature and left to stir overnight. (After stirring at room temperature for 1 hour, a clear yellow solution resulted). The mixture was concentrated in vacuo, and the resulting residue was taken up in 500 ml of ethyl acetate and washed with 5% sodium bcarbonate (3 × 100 ml) and brine (100 ml). The organic layer was dried over sodium sulfate and concentrated to yield a yellow oil with a white precipitate. This material was crystallized from ethyl acetate/hexane (1:1) (four times). The first two crops produced impurities and were discarded; the third and fourth crops yielded 5.25 g of the products as a white solid. This solid was recrystallized from the same solvent to produce 3.76 g of the title compound, melting point 79-81°C (sinters >63°C). Several attempts were made to purify the remaining material by crystallization, but no additional pure crystals were obtained. Therefore, all of the material except the 3.76 g of product was recombined (34 g) and applied to a column of 2 kg of Merck silica gel (230-400 mesh). Elution with ethyl

acetate/hexane (1:1) yielded 5.59 g of a white solid which was crystallized from the same solvent to yield 3.56 g of the product, melting point 79-81 °C (sinters > 63 °C). This material was combined with the 3.76 g of product to yield 7.32 g of product as a white solid. An additional 1.46 g of slightly impure material was obtained from the column, making the total yield before recrystallization 10.81 g.

E) *8-[[1-(Mercaptomethyl)-2-phenylethyl]amino]--8-oxootanoic acid, methyl ester*

A portion of 8-[[1-[(acetylthio)methyl]-2-phenylethyl]amino]-8-oxooctanoic acid, methyl ester (1.898 g, 5.0 mmol) was dissolved in methanol (30 ml) by warming, and then chilled in an ice bath under nitrogen. To this solution was added, dropwise over 10 minutes, 1N sodium hydroxide (5 ml, 1.0 equiv.). The mixture was stirred at 0 °C for 10 minutes and then allowed to warm to room temperature and stirred for 1 hour. The mixture was concentrated in vacuo to remove all methanol. The residue was diluted with water (20 ml), neutralized with 1N hydrochloric acid (5 ml) and extracted with ethyl acetate (3 × 15 ml). These organic layers were combined, washed with water and brine (15 ml each), dried over sodium sulfate and concentrated to yield 1.62 g of a white solid. This material was dissolved in a minimal amount of methanol and adsorbed onto a small amount of Merck silica gel (230-400 mesh) and dried. This material was then applied to a column of 105 g of the same silica and eluted with hexane/acetone (5:2) to yield 1.43 g of the title compound as a white solid, melting point 59-60 °C (sinters > 56 °C).

Analysis Calc'd. for $C_{18}H_{27}NO_3S \cdot 0.38H_2O$:

C 62.77; H 8.13; N 4.07; S 9.31

Found: C 62.77; H 7.90; N 4.03; S 9.40.

*Example 3*

*8-[[1-Mercaptomethyl)-2-phenylethyl]amino]-8-oxooctanoic acid*

8-[[1-(Mercaptomethyl)-2-phenylethyl]amino]-8-oxooctanoic acid, methyl ester (2.72 g, 7.17 mmol; see example 2) was dissolved in methanol (60 ml) and chilled in an ice bath under nitrogen. To this solution was added, dropwise over 10 minutes, 1N sodium hydroxide (22 ml, ~3 equiv.). The mixture was stirred at 0 °C for 10 minutes, warmed to room temperature and left to stir for 3 hours. The mixture was concentrated in vacuo to remove all methanol and the residue was diluted with water (40 ml) and then washed with chloroform (2 × 20 ml). The aqueous layer was acidified with cooling to pH ~1.5 with concentrated hydrochloric acid. The resulting white suspension was extracted with ethyl acetate (3 × 20 ml). These organic layers were combined, washed with water and brine (20 ml each), dried over sodium sulfate and concentrated to yield a white solid, 1.63 g. This material was applied to a column of SilicAr® CC-4 and eluted with chloroform, gradually adding ethyl acetate (10%-40%) yielding 1.55 g of the title compound as a white solid, melting point 90-92 °C (sinters > 87 °C).

Analysis Calc'd. for $C_{17}H_{25}NO_3S$:

C 63.13; H 7.91; N 4.26; S 9.90

Found: C 63.13; H 7.91; N 4.26; S 9.90.

**Claims**

1. A compound having the formula

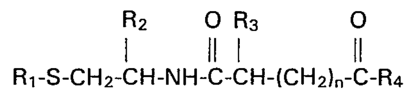

or a pharmaceutically acceptable salt thereof, wherein

O
||
$R_1$ is hydrogen or $R_5$-C-, wherein $R_5$ is alkyl, aryl, arylalkyl, heteroaryl, or (heteroaryl)alkyl;

$R_2$ and $R_3$ are each independently hydrogen, alkyl, (cycloalkyl)alkyl, aryl, arylalkyl, heteroaryl, or (heteroaryl)alkyl;

$R_4$ is hydroxy, alkoxy, arylalkoxy, (heteroaryl)alkoxy, (substituted alkyl)oxy, or $-NR_6R_7$, wherein $R_6$ and $R_7$ are each independently hydrogen, alkyl, substituted alkyl, aryl, arylalkyl, or (heteroaryl)alkyl; and

n is an integer of 1 to 15;

wherein the terms «alkyl», «alkoxy», and «alkylthio» refer to groups having 1 to 7 carbon atoms; the therm «cycloalkyl» refers to cycloalkyl groups having 3, 4, 5, 6 or 7 carbon atoms; the term «aryl» refers to phenyl or phenyl substituted with 1, 2 or 3 alkyl, alkoxy, alkylthio, hydroxy, chlorine, bromine, fluorine, amino, alkylamino, dialkylamino, nitro or trifluoromethyl groups; the term «heteroaryl» refers to 2- or 3-furanyl, 2- or 3-thienyl, 2-, 3- or 4-pyridinyl, 4-imidazolyl or 3-indolyl; and the term «substituted alkyl» refers to alkyl groups substituted with one, or more, hydroxy or $NY_1Y_2$ groups, wherein $Y_1$ and $Y_2$ are the same or different and each is hydrogen or alkyl, $Y_1$ is hydrogen and $Y_2$ is aryl, or $Y_1$ and $Y_2$ together with the nitrogen atom to which they are attached form a heterocyclic group having the formula

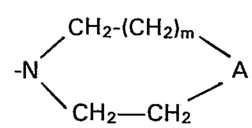

and A is CH-Q, oxygen or N-Q, Q is hydrogen or alkyl and M is 0 or 1.

2. A compound in accordance with claim 1 wherein $R_1$ is hydrogen.

3. A compound in accordance with claim 1

O
||
wherein $R_1$ is $R_5$-C- .

4. A compound in accordance with claim 1, 2 or 3 wherein $R_2$ is hydrogen.

5. A compound in accordance with claim 1, 2 or 3 wherein $R_2$ is alkyl.

6. A compound in accordance with claim 1, 2 or 3 wherein $R_2$ is aryl.

7. A compound in accordance with claim 1, 2 or 3 wherein $R_2$ is arylalkyl.

8. A compound in accordance with claim 1, 2 or 3 wherein $R_2$ is heteroaryl.

9. A compound in accordance with claim 1, 2 or 3 wherein $R_2$ is (heteroaryl)alkyl.

10. A compound in accordance with any one of Claims 1 to 9 wherein $R_3$ is hydrogen.

11. A compound in accordance with any one of Claims 1 to 9 wherein $R_3$ is alkyl.

12. A compound in accordance with any one of Claims 1 to 9 wherein $R_3$ is aryl.

13. A compound in accordance with any one of Claims 1 to 9 wherein $R_3$ is arylalkyl.

14. A compound in accordance with any one of Claims 1 to 9 wherein $R_3$ is heteroaryl.

15. A compound in accordance with any one of Claims 1 to 9 wherein $R_3$ is (heteroaryl)alkyl.

16. A compound in accordance with any one of Claims 1 to 15 wherein $R_4$ is hydroxy.

17. A compound in accordance with any one of Claims 1 to 15 wherein $R_4$ is alkoxy.

18. A compound in accordance with any one of Claims 1 to 15 wherein $R_4$ is $-NR_6R_7$.

19. A compound in accordance with any one of Claims 1 to 18 wherein n is 3 or 5.

20. The compound in accordance with claim 1, 6-[[1-(mercaptomethyl)-2-phenylethyl]amino]-6-oxohexanoic acid.

21. The compound in accordance with claim 1, 8-[[1-(mercaptomethyl)-2-phenylethyl]amino]-8-oxooctanoic acid, methyl ester.

22. The compound in accordance with claim 1, 8-[[1-(mercaptomethyl)-2-phenylethyl]amino]-8-oxooctanoic acid.

**Patentansprüche**

1. Eine Verbindung mit der Formel

$$R_1-S-CH_2-\overset{R_2}{\overset{|}{C}}H-NH-\overset{O}{\overset{\|}{C}}-\overset{R_3}{\overset{|}{C}}H-(CH_2)_n-\overset{O}{\overset{\|}{C}}-R_4$$

oder ein pharmazeutisch verträgliches Salz davon, in der $R_1$ Wasserstoff oder $R_5-\overset{O}{\overset{\|}{C}}-$ bedeutet, wobei $R_5$ Alkyl, Aryl, Arylakyl, Heteroaryl oder (Heteroaryl)-alkyl darstellt;

$R_2$ und $R_3$ jeweils unabhängig voneinander Wasserstoff, Alkyl, (Cycloalkyl)-alkyl, Aryl, Arylalkyl, Heteroaryl oder (Heteroaryl)-alkyl bedeuten;

$R_4$ Hydroxy, Alkoxy, Arylalkoxy, (Heteroaryl)-alkoxy, (substituiertes Alkyl)-oxy oder $-NR_6R_7$ darstellt, wobei $R_6$ und $R_7$ jeweils unabhängig voneinander Wasserstoff, Alkyl, substituiertes Alkyl, Aryl, Arylalkyl oder (Heteroaryl)-alkyl darstellen; und

n eine ganze Zahl im Wert von 1 bis 15 ist; wobei sich die Begriffe «Alkyl», «Alkoxy» und «Alkylthio» auf Reste mit 1 bis 7 Kohlenstoffatomen beziehen, der Begriff «Cycloalkyl» Cycloalkylreste mit 3, 4, 5, 6 oder 7 Kohlenstoffatomen bezeichnet, der Begriff «Aryl» Phenyl oder mit 1, 2 oder 3 Alkyl-, Alkoxy-, Alkylthio-, Hydroxy, Chlor-, Brom-, Fluor-, Amino-, Alkylamino-, Dialkylamino-, Nitro- oder Trifluormethylgruppen substituiertes Phenyl bedeutet, der Begriff «Heteroaryl» sich auf 2- oder 3-Furanyl, 2- oder 3-Thienyl, 2-, 3- oder 4-Pyridinyl, 4-Imidazolyl oder 3-Indolyl bezieht, und der Begriff «substituiertes Alkyl» mit einer oder mehreren Hydroxy- oder $-NY_1Y_2$-Gruppen substituierte Alkylreste bedeutet, wobei $Y_1$ und $Y_2$ gleich oder verschieden sind und jeweils Wasserstoff oder Alkyl bedeuten, $Y_1$ Wasserstoff ist und $Y_2$ Aryl bedeutet, oder $Y_1$ und $Y_2$ zusammen mit dem Stickstoffatom, an das sie gebunden sind, einen heterocyclischen Rest der Formel

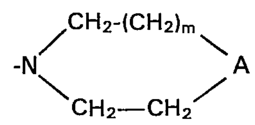

bilden, und A CH-Q, Sauerstoff oder N-Q bedeutet, Q Wasserstoff oder Alkyl ist und m den Wert 0 oder 1 hat.

2. Verbindung nach Anspruch 1, in der $R_1$ Wasserstoff ist.

3. Verbindung nach Anspruch 1, in der $R_1$ $-R_5-\overset{O}{\overset{\|}{C}}-$ bedeutet.

4. Verbindung nach Anspruch 1, 2 oder 3, in der $R_2$ Wasserstoff bedeutet.

5. Verbindung nach Anspruch 1, 2 oder 3, in der $R_2$ Alkyl bedeutet.

6. Verbindung nach Anspruch 1, 2 oder 3, in der $R_2$ Aryl bedeutet.

7. Verbindung nach Anspruch 1, 2 oder 3, in der $R_2$ Arylalkyl bedeutet.

8. Verbindung nach Anspruch 1, 2 oder 3, in der $R_2$ Heteroaryl bedeutet.

9. Verbindung nach Anspruch 1, 2 oder 3, in der $R_2$ (Heteroaryl)-alkyl bedeutet.

10. Verbindung nach einem der Ansprüche 1 bis 9, in der $R_3$ Wasserstoff bedeutet.

11. Verbindung nach einem der Ansprüche 1 bis 9, in der $R_3$ Alkyl bedeutet.

12. Verbindung nach einem der Ansprüche 1 bis 9, in der $R_3$ Aryl bedeutet.

13. Verbindung nach einem der Ansprüche 1 bis 9, in der $R_3$ Arylalkyl bedeutet.

14. Verbindung nach einem der Ansprüche 1 bis 9, in der $R_3$ Heteroaryl bedeutet.

15. Verbindung nach einem der Ansprüche 1 bis 9, in der $R_3$ (Heteroaryl)-alkyl bedeutet.

16. Verbindung nach einem der Ansprüche 1 bis 15, in der $R_4$ Hydroxy bedeutet.

17. Verbindung nach einem der Ansprüche 1 bis 15, in der $R_4$ Alkoxy bedeutet.

18. Verbindung nach einem der Ansprüche 1 bis 15, in der $R_4$ $-NR_6R_7$ bedeutet.

19. Verbindung nach einem der Ansprüche 1 bis 18, in der n den Wert 3 oder 5 hat.

20. Verbindung nach Anspruch 1, nämlich 6-[[1-(Mercaptomethyl)-2-phenyläthyl]-amino]-6-oxohexansäure.

21. Verbindung nach Anspruch 1, nämlich 8-[[1-(Mercaptomethyl)-2-phenyläthyl]-amino]-8-oxooctansäure-methylester.

22. Verbindung nach Anspruch 1, 8-[[1-Mercaptomethyl)-2-phenyläthyl]-amino]-8-oxooctansäure.

## Revendications

1. Composé ayant pour formule

$$\begin{array}{l} \quad R_2 \quad\quad O\ R_3 \quad\quad\quad\quad O \\ \quad | \quad\quad\ \ \| \ \ | \quad\quad\quad\quad\quad \| \\ R_1\text{-}S\text{-}CH_2\text{-}CH\text{-}NH\text{-}C\text{-}CH\text{-}(CH_2)_n\text{-}C\text{-}R_4 \end{array}$$

ou sel pharmaceutiquement acceptable de celui-ci, formule dans laquelle

$R_1$ est un atome d'hydrogène ou un groupement de formule $R_5\text{-}\overset{\overset{O}{\|}}{C}\text{-}$ dans laquelle $R_5$ est un radical alkyle, aryle, arylalkyle, hétéroaryle ou (hétéroaryl)alkyle;

$R_2$ ou $R_3$ sont chacun, indépendamment, un atome d'hydrogène ou un radical alkyle, (cycloalkyl)alkyle, aryle, arylalkyle, hétéroaryl)alkyle;

$R_4$ est un groupement hydroxy, alcoxy, arylalcoxy, (hétéroaryl)alcoxy , (alkyl substitué)oxy, ou de formule $-NR_6R_7$ dans laquelle $R_6$ et $R_7$ sont chacun, indépendamment, un atome d'hydrogène ou un radical alkyle éventuellement substitué, aryle, arylalkyle ou (hétéroaryl)alkyle; et

n est un nombre entier de 1 à 15;

où les termes «alkyle», «alcoxy» et «alkylthio» désignent des groupements ayant de 1 à 7 atomes de carbone; le terme «cycloalkyle» désigne des radicaux cycloalkyle ayant 3, 4, 5, 6 ou 7 atomes de carbone; le terme «aryle» désigne un radical phényle éventuellement substitué par 1, 2 ou 3 radicaux alkyle, alcoxy ou alkylthio, groupement hydroxy, atomes de chlore, de brome ou de fluor, ou groupements amine, alkylamine, dialkylamine, nitro ou trifluorométhyle; le terme «hétéroaryle» désigne un radical 2- ou 3-furanyle, 2- ou 3-thiényle, 2-, 3- ou 4-pyridinyle, 4-imidazolyle ou 3-indolyle; et le terme «alkyle substitué» désigne des radicaux alkyle substitués par un ou plusieurs groupements hydroxy ou $NY_1Y_2$ où $Y_1$ et $Y_2$ sont identiques ou différents et sont chacun un atome d'hydrogène ou un radical alkyle, $Y_1$ est un atome d'hydrogène et $Y_2$ est un radical aryle, ou bien $Y_1$ et $Y_2$ forment avec l'atome d'azote auquel ils sont fixés un groupement hétérocyclique ayant pour formule

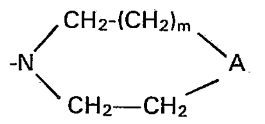

et A est CH-Q, un atome d'oxygène ou N-Q, Q est un atome d'hydrogène ou un radical alkyle et m est égal à 0 ou 1.

2. Composé selon la revendication 1, dans la formule duquel $R_1$ est un atome d'hydrogène.

3. Composé selon la revendication 1, dans la formule duquel $R_1$ est $R_5\text{-}\overset{\overset{O}{\|}}{C}\text{-}$.

4. Composé selon la revendication 1, 2 ou 3, dans la formule duquel $R_2$ est un atome d'hydrogène.

5. Composé selon la revendication 1, 2 ou 3, dans la formule duquel $R_2$ est un radical alkyle.

6. Composé selon la revendication 1, 2 ou 3, dans la formule duquel $R_2$ est un radical aryle.

7. Composé selon la revendication 1, 2 ou 3, dans la formule duquel $R_2$ est un radical arylalkyle.

8. Composé selon la revendication 1, 2 ou 3, dans la formule duquel $R_2$ est un radical hétéroaryle.

9. Composé selon la revendication 1, 2 ou 3, dans la formule duquel $R_2$ est un radical (hétéroaryl)alkyle.

10. Composé selon l'une quelconque des revendications 1 à 9, dans la formule duquel $R_3$ est un atome d'hydrogène.

11. Composé selon l'une quelconque des revendications 1 à 9, dans la formule duquel $R_3$ est un radical alkyle.

12. Composé selon l'une quelconque des revendications 1 à 9, dans la formule duquel $R_3$ est un radical aryle.

13. Composé selon l'une quelconque des revendications 1 à 9, dans la formule duquel $R_3$ est un radical arylalkyle.

14. Composé selon l'une quelconque des revendications 1 à 9, dans la formule duquel $R_3$ est un radical hétéroaryle.

15. Composé selon l'une quelconque des revendications 1 à 9, dans la formule duquel $R_3$ est un radical (hétéroaryk)alkyle.

16. Composé selon l'une quelconque des revendications 1 à 15, dans la formule duquel $R_4$ est un groupement hydroxy.

17. Composé selon l'une quelconque des revendications 1 à 15, dans la formule duquel $R_4$ est un radical alcoxy.

18. Composé selon l'une quelconque des revendications 1 à 15, dans la formule duquel $R_4$ est $-NR_6R_7$.

19. Composé selon l'une quelconque des revendications 1 à 18, dans la formule duquel n est égal à 3 ou 5.

20. Composé selon la revendication 1, qui est l'acide 6-[[1-(mercaptométhyl)-2-phényléthyl]-amino]-6-oxohexanoïque.

21. Composé selon la revendication 1, qui est l'ester méthylique de l'acide 8-[[1-(mercaptométhl)-2-phényléthyl]amino]-8-oxo-octanoïque.

22. Composé selon la revendication 1, qui est l'acide 8-[[1-mercaptométhyl)-2-phényléthyl]-amino]-8-oxo-octanoïque.